# EUROPEAN PATENT APPLICATION

(11) **EP 4 755 216 A2**
(43) Date of publication of application: **10.06.2026**
(21) Application number: 26174743.0
(22) Date of filing: 17.03.2020
(51) Int. Cl.: A24F 40/44

(54) **AEROSOL DELIVERY SYSTEM**

(30) Priority: 21.03.2019 EP 19164467
(62) Divisional of application: 24207311.2
(71) Applicant: Imperial Tobacco Limited, Bristol BS3 2LL (GB)
(72) Inventor: LORD, Chris, Bristol, BS3 2L (GB); SUDLOW, Thomas, Bristol, BS3 2L (GB); ILLIDGE, Ben, Bristol, BS3 2LL (GB); MADDEN, Alfred, Bristol, BS3 2LL (GB); ASTBURY, Ben, Bristol, BS3 2L (GB)
(74) Representative: Mewburn Ellis LLP

(57) **Abstract**

An aerosol delivery system has a fluid transfer article with a first region for holding an aerosol precursor, and a wick which extends from the reservoir and makes abutting unbonded contact with a heating region of a heater of the aerosol-delivery system. There may be a seal closing the reservoir, with the wick extending through the seal so that aerosol precursor may pass from the reservoir through the wick to the heating surface. When the heater is active, that aerosol precursor will be heated to form an aerosol or vapour. When the user sucks or inhales through the aerosol delivery system, air flows through the air-flow pathway across the heating surface of the heater, so that aerosol passes from the heater and/or wick to the user in the air-flow. The heating region of the heater is flexible and therefore deforms due to the contact between itself and the wick ensuring good contact therebetween. The fluid-transfer article and the heater are separable, to allow the fluid-transfer article to be replaced.

## Description

### Field of the Invention

The present invention relates to an aerosol delivery system, and an aerosol-generation apparatus for an aerosol delivery system. The present invention preferably relates to an aerosol delivery system including a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette^{®} Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, inter alia, as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

### Summary of the Invention

At its most general, the present invention proposes that an aerosol-generation apparatus has a heater which has a heating region in abutting unbonded contact with a wick of a fluid-transfer article. The fluid-transfer article is separable (e.g. removable) from the heater, and includes a reservoir for holding an aerosol precursor and a wick. The wick transfers the aerosol precursor from the reservoir towards the heating region of the heater. The heating region is flexible, so that it may deform due to the contact between the heating region and the wick. This allows it to conform to the shape of the wick.

Thus, when the fluid-transfer article is in place in the aerosol-generation apparatus, aerosol precursor may pass through the wick to the heating region of the heater, to be heated thereby when the heater is active. This forms a vapour or vapour/aerosol mixture, which may then pass to the user in an air flow. The flexibility of the heating region means that, when the fluid-transfer article is inserted into the rest of the aerosol-generation apparatus, the heating region will conform to the wick which it contacts. The separability of the fluid-transfer article means that the fluid-transfer article can be removed (e.g. when the aerosol precursor has been used up) and replaced.

Thus, according to the present invention, there may be provided an aerosol-generation apparatus comprising a heater and a fluid-transfer article, the fluid-transfer article comprising a reservoir for holding an aerosol precursor and a wick arranged and configured to receive said aerosol precursor from said reservoir, the wick making abutting unbonded contact with a heating region of said heater, wherein said heating region of said heater is flexible, thereby to be deformable due to said contact between said wick and said heating region, and wherein said fluid-transfer article and said heater are separable.

Preferably, the heating region is resilient. This may enable the abutting unbonded contact between the heating region and the wick to be a resilient contact.

The wick may be U-shaped with the base of the U-shape making the unbonded contact with the heating region.

Preferably, there will be a sealing member sealing the reservoir, to prevent leakage of aerosol precursor. The wick may then extend through the sealing member into the reservoir. This enables it to receive aerosol precursor from the reservoir and to pass that aerosol precursor to the heating surface. Where such a sealing member is used, and the flexible wick is U-shaped, both arms of the U-shape may extend through the sealing member.

The wick may be relatively rigid. For example, it may be made of a porous polymeric material. Optionally, however, the wick itself is flexible, and may then be made of a cord material, although other materials are possible. When the wick is flexible, it is desirable that it is less flexible than the heating surface, so that it is the heating surface which flexes to conform to the wick.

There will normally be an air-flow pathway past the heating region, to enable air to pass to the user. When the heater is active, the air flowing along the air-flow pathway will receive vapour and/or vapour/aerosol mixture from the heated wick, which vapour or mixture can pass to the user with the air flow.

As mentioned above the fluid-transfer article is normally separable from the rest of the aerosol-generation apparatus. There may therefore be provided a carrier with a first housing containing the reservoir and supporting the wick. A second housing supporting the heater may also be provided, with those housings then separable. In such an arrangement, the housing containing the reservoir may have an outlet, and the housing supporting the heater have an inlet, so that the air-flow pathway extends to the inlet and outlet to enable air to flow to the user.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1****.** is a perspective view illustration of a system for aerosol delivery according to one or more embodiments of the present invention;
**Figure 2****.** is a cross-section side view illustration of part of an apparatus of the system for aerosol delivery of Figure 1;
**Figure 3****.** is a cross-section side view illustration of the system and apparatus for aerosol delivery of Figure 1;
**Figure 4****.** is a perspective view illustration of an aerosol carrier for use in the system for aerosol delivery according to one or more embodiments of the present invention;
**Figure 5****.** is a cross-section side view of elements of an aerosol carrier and a part of an apparatus of the system for aerosol delivery according to one or more embodiments of the present invention;
**Figure 6****.** shows in more detail the configuration of the heater in the arrangement of Figure 5;
**Figure 7****.** is a cross-section side view of aerosol carrier according to one or more embodiments of the present invention;
**Figure 8****.** is a perspective cross-section side view of the aerosol carrier of Figure 7, and;
**Figure 9****.** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

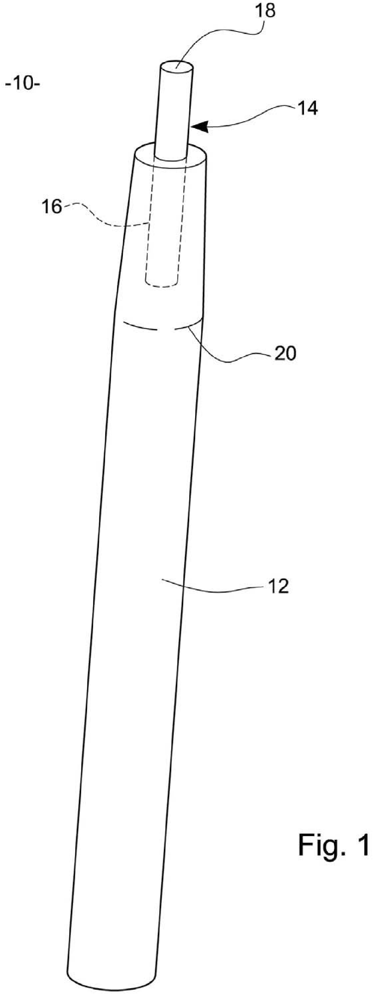

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn to the wick of a fluid-transfer article located within a housing of the aerosol carrier cartridge 14 during use. Optionally, these apertures may be perforations in the housing of the apparatus 12.

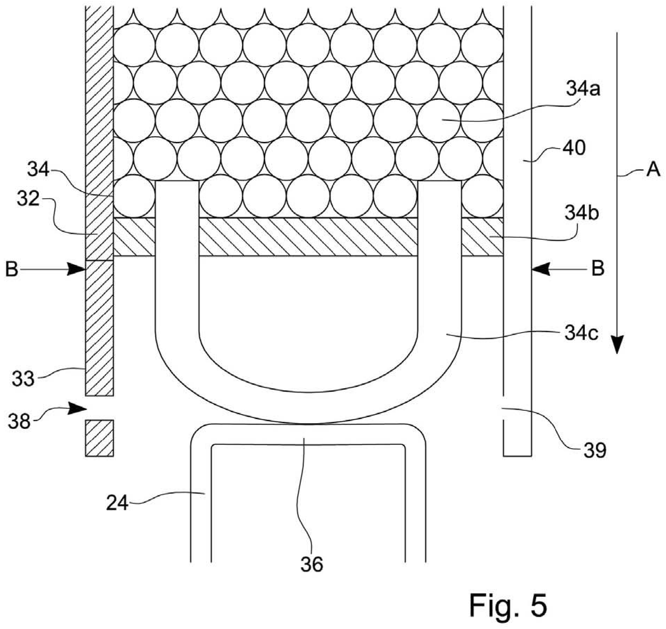

A fluid-transfer article (not shown in Figure 1, but described hereinafter with reference to Figs. 5 to 8 is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow to a wick of the fluid-transfer article. As air passes the wick of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

Part of the fluid-transfer article 34 may comprise a porous material where pores of the porous material hold, contain, carry, or bear the aerosol precursor material. In particular, the porous material of the fluid-transfer article is a porous polymer material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET). All such materials may be described as heat resistant polymeric wicking material in the context of the present invention.

Alternatively, the fluid-transfer article 34 may have an open reservoir for aerosol precursor, with a suitable seal to prevent leakage.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

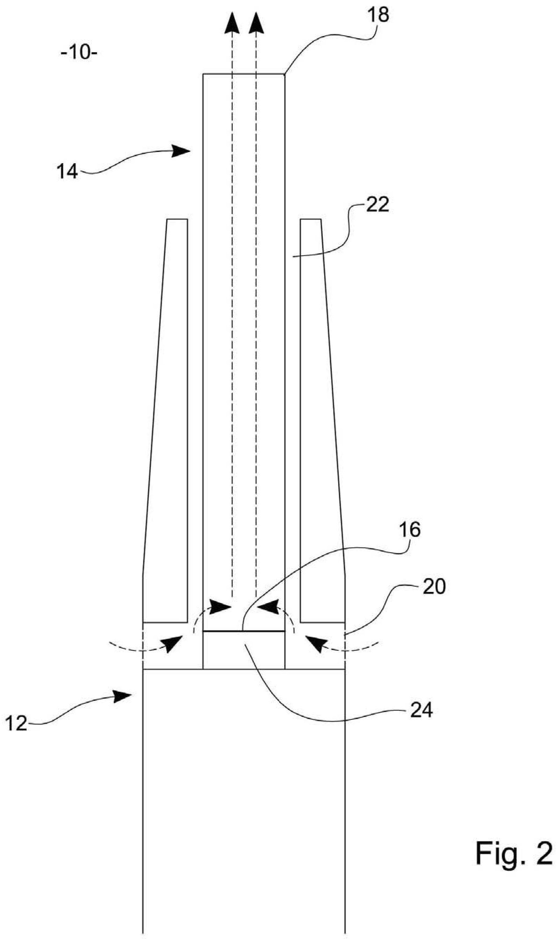

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprises a heater 24, which has a heating region in contact with part of a wick of the fluid-transfer article (not shown in Figure 2) of the aerosol carrier 14 when an aerosol carrier 14 is located within the receptacle 22.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the heating region of the heater 24 and around the wick. Heat from the heating region of the heater 24, which is in contact with the wick of the fluid-transfer article, causes vaporisation of aerosol precursor material in the wick of the fluid-transfer article and an aerosol is created in the air flowing over the heating surface. Thus, through the application of heat, aerosol is released or liberated from the wick of the fluid-transfer article, and is drawn from the material of the aerosol carrier unit by the air flowing past the heating region of the heater and is transported in the air flow to the user via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14, and in particular the wick thereof, is heated by the heater 24. As a user sucks or inhales on second end 18 of the aerosol carrier 14, the aerosol released from the wick of the fluid-transfer article and entrained in the air flowing past the heating region of the heater 24 is drawn through the outlet conduits (not shown) in the housing of the aerosol carrier 14 towards the second end 18 and onwards into the user's mouth.

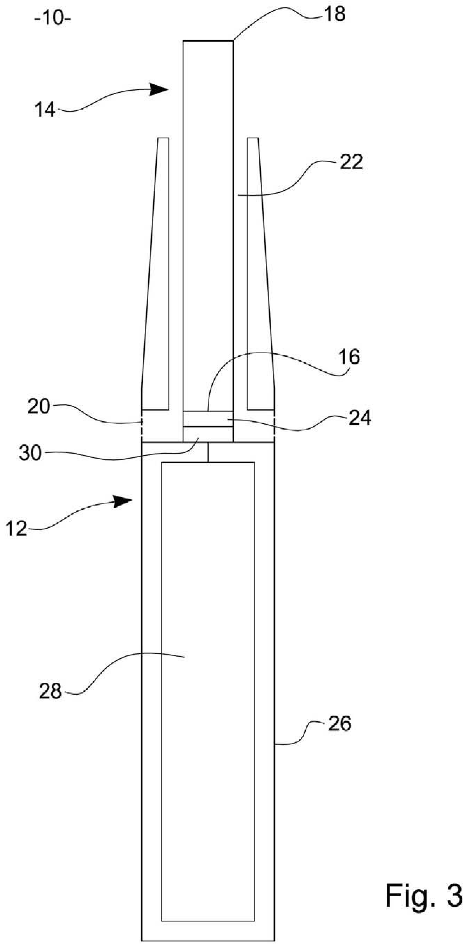

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heater 24 heats the wick of the fluid-transfer article (not shown in Figure 3) of aerosol carrier 14. This heating process initiates (and, through continued operation, maintains) release of vapour and/or an aerosol from the activation surface of the fluid-transfer article. The vapour and/or aerosol formed as a result of the heating process is entrained into a stream of air being drawn past the heating region of the heater (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

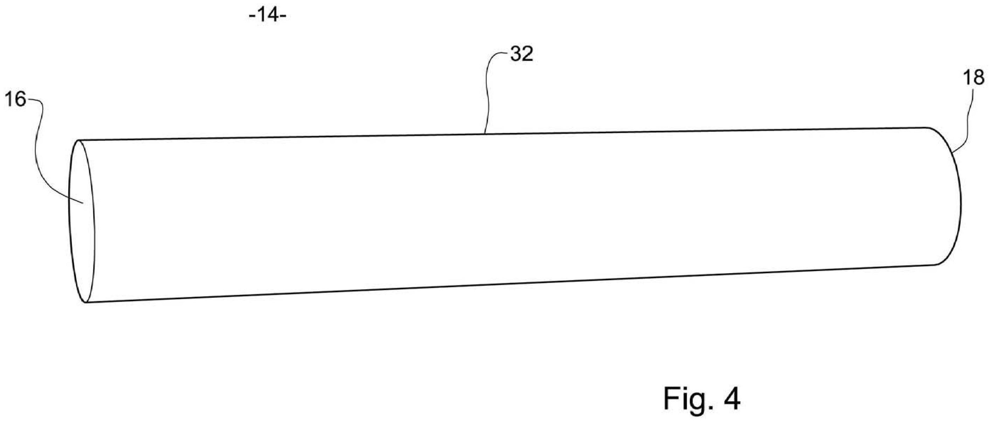

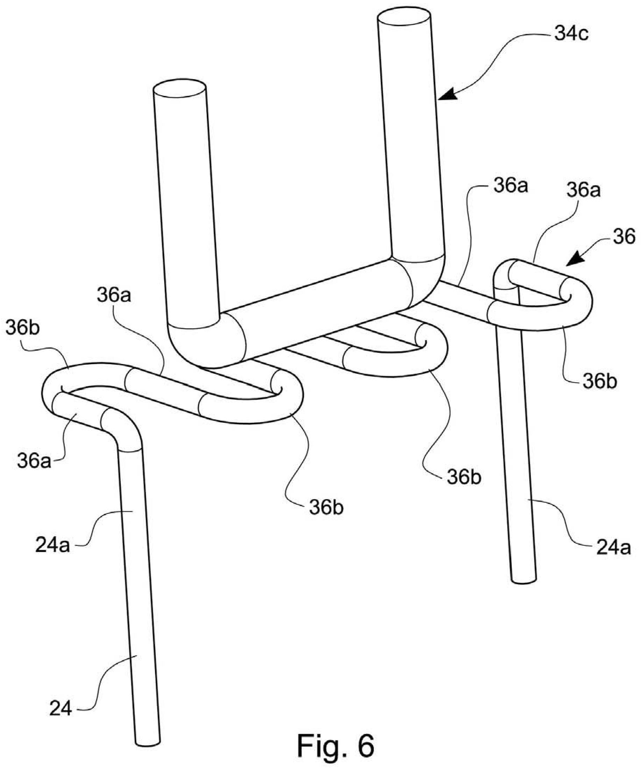

Figures 4 to 6 schematically illustrate the aerosol carrier 14 in more detail (and, in Figures 5 and 6, features within the receptacle in more detail). Figure 4 illustrates an exterior of the aerosol carrier 14, Figure 5 illustrates internal components of the aerosol carrier 14 in one optional configuration, and Fig. 6 illustrates a possible configuration of the heater 24.

Fig. 4 illustrates the exterior of the aerosol carrier 14, which comprises housing 32 for housing said fluid-transfer article (not shown). The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12, in in one embodiment of the invention.

Further components not shown in Figure 5 comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figure 5, the aerosol carrier is shown as comprising the fluid-transfer article 34 located within housing 32. The fluid transfer article 34 comprises a first region 34a holding an aerosol precursor. In one or more arrangements, the first region of 34a of the fluid transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a. The material forming the first region of 34a may comprise a porous structure, whose pore diameter size varies between one end of the first region 34a and another end of the first region 34a. The pore diameter size may decrease from a first end remote from heater 24 (the upper end is as shown in the figure) to a second end. The change in pore size in the first region 34a may be gradual rather than step-wise. This configuration of pores having a decreasing diameter size can provide a wicking effect, which can serve to draw fluid through the first region 34a.

Particular examples of material suitable for the first region 34a of the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET).

Alternatively, the first region 34a may be a simple liquid reservoir in the form of an empty tank for the receipt of liquid aerosol precursor, rather than porous material for holding the aerosol precursor.

The fluid-transfer article also comprises a second region 34b, acting as a seal for the first region 34a. This is particularly important if first region 34a is a tank containing liquid. The second region 34b thus prevents unwanted escape of aerosol precursor from the first region 34a.

As mentioned above, the fluid-transfer article also includes a wick 34c. In the arrangement shown in Figure 5, the wick 34c is flexible and U-shaped, with the arms of the U-shape extending through the second region 34b into the first region 34a. The wick 34c is absorbent, so that its ends absorb aerosol precursor from the first region 34a. For example, the wick 34c may be of a cord material. The aerosol precursor will pass from the first region 34a through and along the wick 34c towards the base of the U-shape of the wick 34c. As illustrated in Figure 5, the base of the U-shape of the flexible wick 34c is in contact with the heater 24 at a heating region. Thus, when the heater 24 is active, heat will be transferred from the heater 24 to the wick 34c, thereby heating the aerosol precursor which has been absorbed by the wick 34c. The heating of the wick 34c will release aerosol precursor from the wick 34c, as a vapour and/or a mixture of vapour and aerosol.

The configuration of the wick 34c is not limited to arrangement shown in Figure 5. For example, it may be a relatively rigid body which forms an end for the region 34a. The second region 34b may then not be necessary. In such a case, the wick may be made of a porous polymer material, e.g. those referred to above as heat resistant polymeric wicking materials.

Figure 5 also illustrates an opening 38 in a further housing 33, which opening 38 is in communication with the air-intake apertures 20. A further opening 39 communicates with a duct 40 within the housing 32, which duct 40 communicates with the second end 18. The further housing 33 may be integral with the housing 26 containing the electrical energy supply 28.

There is thus a fluid-flow path for air (hereinafter referred to as an air-flow pathway) between openings 38 and 39, linking the apertures 20 and the second end 18 of the aerosol carrier. When the user sucks or inhales, air is drawn along the air-flow pathway, past a heating region 36 of the heater 24.

One or more droplets of the aerosol precursor will be released from the wick 34c as it is heated, to release vapour or a mixture of aerosol and vapour into the air flowing in the air-flow pathway between the openings 38, 39. The vapour or mixture passes, as the user sucks and inhales, to the second end 18.

Figure 6 shows an embodiment of the heater 24 in more detail. As illustrated, the heater has a heating region 36 comprising alternating straight sections 36a and U-shaped sections 36b, so that heating region 36 is convoluted. This enables good contact to be made between the heating region 36 and the wick 34c, whilst allowing for variations in their relative positions. Hence, when the housings 32 and 33 are joined, the contact between the wick 34c and the heating region 36 is not dependent on the precise positioning or orientation of the housings 32 and 33.

The heating region 36 is supported on upright sections 24a of the heater 24, which upright sections 24a may extend from the coupling 30 shown in Figure 3.

The straight and U-shaped sections 36a, 36b of the heating region 36 may be arranged so that, in the absence of deformation, they lie in a plane, whereby the heating region 36 is flat prior to contact with the wick 34c. That contact may then deform the heating region 36 so that it becomes somewhat concave, thereby conforming at least partially to the shape of the wick 34c. Alternatively, the heating region 36 may be curved, either concave or convex, in the absence of deformation. For example, a convex arrangement, curved towards the wick 34c, may assist in ensuring good contact between the wick 34c and the heating region 36.

It is possible for the whole of the heating region 36 to be flexible, so both the straight sections 36a and the U-shaped sections 36b are flexible. Alternatively, however, only the U-shaped sections 36b may be flexible, and the straight sections 36a may be rigid, as this would still allow the heating region 36 to conform generally to the wick 34c. The opposite arrangement would also be possible, namely rigid U-shaped sections 36b and flexible straight sections 36a. In either case, it is desirable that the flexibility of the heating region 36 of the heater 24 is a resilient flexibility, both to allow the heating region 36 to return to its original shape if the fluid-transfer article, and hence the wick, is removed from the rest of the apparatus, and also because the resilience will ensure that contact is not lost, e.g. due to impact on, or shaking of, the apparatus.

The deformation of the heating region 36, due to its flexibility, will have the effect of increasing surface area contact between the heating region 36 and the wick 34c, which may improve the rate of vaporisation of the aerosol precursor. This may be improved further if the wick 34c is itself somewhat flexible.

In the illustrative examples of Figure 5, the first region 34a of the fluid-transfer article 34 is located at an "upstream" end of the fluid-transfer article 34 and the flexible wick 34c is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

In the arrangement of Figure 5, the housing 32 contains the first and second parts 34a, 34b of the fluid-transfer article, and also supports the wick 34c. The heater 24 is supported by the further housing 33 which has the openings 38 and 39 therein, Housings 32 and 33 are separable, for example along the line B-B in Figure 5. Thus, the housing 32 and hence the fluid-transfer article 34 may be removed from the rest of the structure, for example when the aerosol precursor therein has been depleted. The aerosol precursor may be re-filled, or the carrier 14 replaced with another filled one. As mentioned above, the heating region 36 is flexible, and is preferably resilient. Then, when the carrier 14 is in place, and the housing 32 is in the position shown in Figure 5 relative to the housing 33, the contact between the wick 34c and the heating region 36 will be resilient, so that the heating region 36 is biased into contact with the wick 34c to ensure good heat transfer to the wick 34c.

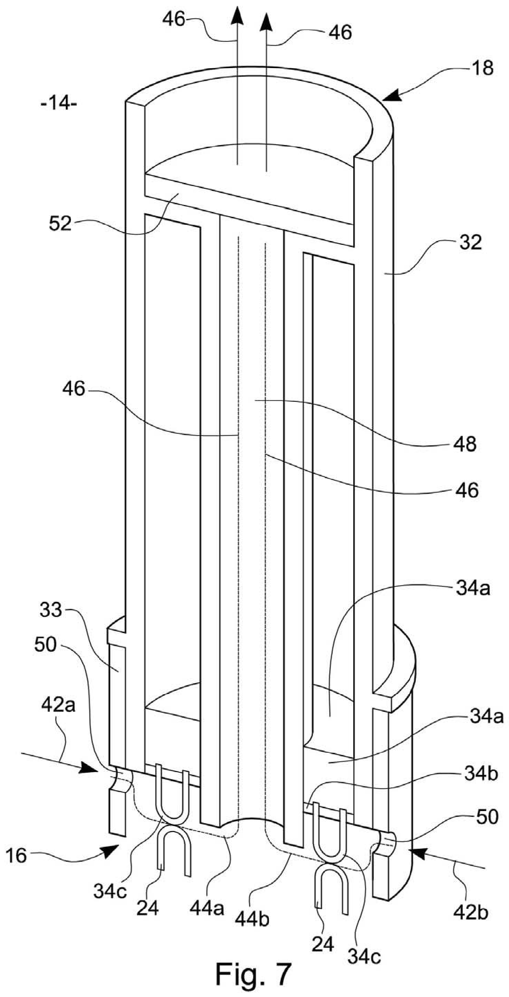

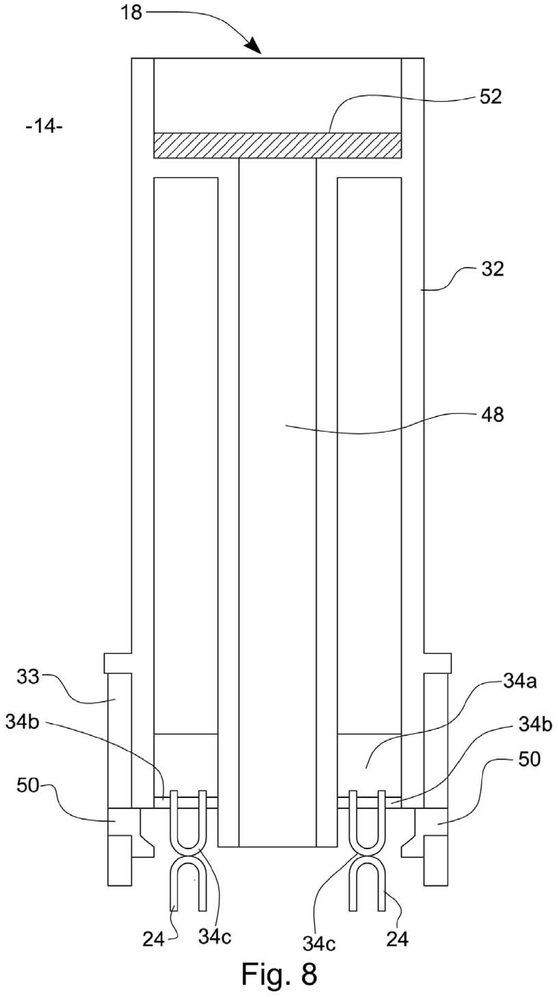

In the arrangements shown in Figures 5 and 6, the apertures 38, 39 are on opposite sides of the housing 32. Figures 7 and 8 show an alternative configuration, in which the fluid-transfer article is annular, and the first and second regions 34a, 34b are also annular. In Figures 7 and 8, the second region 34b is illustrated in a position corresponding to that shown in Figures 5 and 6, where it is spaced from the heating region 36 of the heater 24. This enables the air flow in the apparatus to be illustrated. Thus, Figures 7 and 8 illustrate an aerosol carrier 14 according to one or more possible arrangements in more detail. Figure 7 is a cross-section side view illustration of the aerosol carrier 14 and Figure 8 is a perspective cross-section side view illustration of the aerosol carrier 14.

As can be seen from Figures 7 and 8, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 7 and 8, the fluid-transfer article 34 is an annular shaped element located around the fluid communication pathway 48. A plurality of wicks 34c may be provided around the fluid communication pathway 48, or there may be a single wick in the form of a toroid with a gap therein to form arms which pass through the second region 34b of the fluid-transfer article and extend into the first part 34a to receive aerosol precursor therefrom. Similarly, there may be a plurality of heaters 24 around the fluid communication pathway 48, each being generally similar to the arrangement shown in Figure 6, or there may be single heater with a toroidal heating region 36.

In the walls of the housing 33, there are provided inlet apertures 50 to provide a fluid communication pathway for an incoming air stream to reach the fluid-transfer article 34, and particularly the air-flow pathway defined past the heating region 36.

In the illustrated example of Figures 7 and 8, the aerosol carrier 14 further comprises a filter element 52. The filter element 52 is located across the fluid communication pathway 48 such that an outgoing air stream passing through the fluid communication pathway 48 passes through the filter element 52.

With reference to Figure 8, when a user sucks on a mouthpiece of the apparatus (or on the second end 18 of the aerosol carrier 14, if configured as a mouthpiece), air is drawn into the carrier through inlet apertures 50 extending through walls in the housing 33.

An incoming air stream 42a from a first side of the aerosol carrier 14 is directed to a first side of the second part 34b of the fluid-transfer article 34 (e.g. via a gas communication pathway within the housing of the carrier). An incoming air stream 42b from a second side of the aerosol carrier 14 is directed to a second side of the second part 34b of the fluid-transfer article 34 (e.g. via a gas communication pathway within the housing of the carrier). When the incoming air stream 42a from the first side of the aerosol carrier 14 reaches the first side of the second part 34b, the incoming air stream 42a from the first side of the aerosol carrier 14 flows past the heating region 36. Likewise, when the incoming air stream 42b from the second side of the aerosol carrier 14 reaches the second side of the second part 34b, the incoming air stream 42b from the second side of the aerosol carrier 14 flows past the heating region 36. The air streams from each side are denoted by dashed lines 44a and 44b in Figure 8. As these air streams 44a and 44b flow, aerosol precursor in the flexible wick 34c or on the heating region 36 is entrained in air streams 44a and 44b.

In use, the heater 24 of the apparatus 12 raises a temperature of the wick 34c, to a sufficient temperature to release, or liberate, captive substances (i.e. the aerosol precursor) to form a vapour and/or aerosol, which is drawn downstream. As the air streams 44a and 44b continue their passages, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

In Figures 7 and 8, the housing 32 is separable from the housing 33, as in the arrangements of Figures 5 and 6. This enables the carrier 14, hence the fluid-transfer article 34 to be removed from the rest of the structure and a depleted aerosol precursor to be replaced.

In any of the embodiments described above the second region 34b may have a thickness of less than 5mm. In other embodiments it may have a thickness of: less than 3.5mm, less than 3mm, less than 2.5mm, less than 2mm, less than 1.9mm, less than 1.8mm, less than 1.7mm, less than 1.6mm, less than 1.5mm, less than 1.4mm, less than 1.3mm, less than 1.2mm, less than 1.1mm, less than 1mm, less than 0.9mm, less than 0.8mm, less than 0.7mm, less than 0.6mm, less than 0.5mm, less than 0.4mm, less than 0.3mm, less than 0.2mm, or less than 0.1mm.

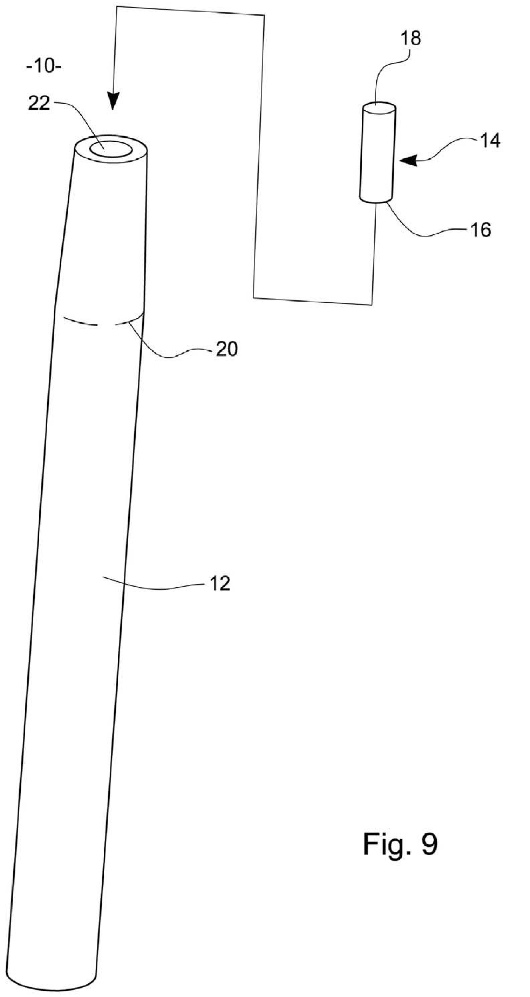

Figure 9 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

Features of embodiments of the invention are set out in the following paragraphs:
Clause 1: An aerosol-generation apparatus comprising a heater and a fluid-transfer article, the fluid-transfer article comprising a reservoir for holding an aerosol precursor and a wick arranged and configured to receive said aerosol precursor from said reservoir, the wick making abutting unbonded contact with a heating region of said heater, wherein said heating region of said heater is flexible, thereby to be deformable due to said contact between said wick and said heating region, and wherein said fluid-transfer article and said heater are separable.
Clause 2: An aerosol-generation apparatus according to clause 1, wherein said at least said heating region of said heater is resilient, whereby said abutting unbonded contact between the heating region and the wick is a resilient contact.
Clause 3: An aerosol-generation apparatus according to clause 1 or clause 2, wherein said heating region of said heater is convoluted.
Clause 4: An aerosol-generation apparatus according to any one of the preceding clauses, wherein said wick is U-shaped, with the base of the U-shape making said unbonded contact with said heating region.
Clause 5: An aerosol-generation apparatus according any one of the preceding clauses, wherein said fluid-transfer article includes a sealing member sealing said reservoir, said wick extending through said sealing member.
Clause 6: An aerosol-generation apparatus according to clause 5 as dependent on clause 4, wherein both arms of said U-shape extend through said sealing member.
Clause 7: An aerosol-generation apparatus according to any of the preceding clauses, wherein the wick is flexible.
Clause 8: An aerosol-generation apparatus according to clause 7, wherein said wick is made of a cord material.
Clause 9: An aerosol-generation apparatus according to any one of the preceding clauses, wherein said heating surface of said heater is located in an air-flow pathway.
Clause 10: An aerosol-delivery system comprising an aerosol-generation apparatus according to any one of the preceding clauses, a carrier having a first housing containing said reservoir and supporting said wick, and a second housing containing said heater, the first and second housings being separable.
Clause 11: An aerosol-delivery system according to clause 10 as dependent on clause 9, wherein said second housing has an inlet, said first housing has an outlet, and said air-flow pathway extends to said inlet and said outlet.

## Claims

1. An aerosol-generation apparatus (12) comprising a heater (24) , said heater having a heating region (36) configured to make abutting unbonded contact with a wick of a fluid-transfer article (34), wherein said fluid-transfer article (34) and said heater (24) are separable, and **characterized in that** said heating region (36) of said heater (24) is flexible, thereby to be deformable due to said contact between said wick (34c) and said heating region (36).

2. The aerosol-generation apparatus (12) according to claim 1, wherein said at least said heating region (36) of said heater (24) is resilient, whereby said abutting unbonded contact between the heating region (36) and the wick (34c) is a resilient contact.

3. The aerosol-generation apparatus (12) according to claim 1 or 2, wherein said heating region of said heater is convoluted.

4. The aerosol-generation apparatus (12) according to claim 3, wherein the heating region comprises alternating straight sections (36a) and U-shaped sections (36b).

5. The aerosol-generation apparatus (12) according to claim 4, wherein the straight sections and U-shaped sections are arranged such that, in the absence of deformation, they lie in a plane, whereby the heating region (36) is flat prior to said contact with the wick (34c).

6. The aerosol-generation apparatus (12) according to any one of claims 1 to 4, wherein the heating region (36) is curved in the absence of deformation.

7. The aerosol-generation apparatus according to any preceding claim, further comprising the fluid transfer article (34), the fluid-transfer article (34) comprising a reservoir for holding an aerosol precursor and the wick (34c), the wick being arranged and configured to receive said aerosol precursor from said reservoir.

8. An aerosol-generation apparatus (12) according to claim 7, wherein said wick (34c) is U-shaped, with the base of the U-shape making said unbonded contact with said heating region (36).

9. An aerosol-generation apparatus (12) according to claim 7 or 8, wherein said fluid-transfer article (34) includes a sealing member (34b) sealing said reservoir, said wick (34c) extending through said sealing member (34b).

10. An aerosol-generation apparatus (12) according to claim 9 as dependent on claim 8, wherein both arms of said U-shape extend through said sealing member (34b).

11. An aerosol-generation apparatus (12) according to any one of claims 7 to 10, wherein the wick (34c) is flexible, optionally wherein said wick (34c) is made of a cord material.

12. An aerosol-generation apparatus (12) according to any one of claims 7 to 11, wherein the wick is less flexible than the heating region.

13. An aerosol-generation apparatus (12) according to any one of the preceding claims, wherein said heating region of said heater is located in an air-flow pathway.

14. An aerosol-delivery system (10) comprising an aerosol-generation apparatus (12) according to any one of claims 7 to 13, a carrier (14) having a first housing (32) containing said reservoir and supporting said wick (34c), and a second housing (33) containing said heater (24), the first and second housings (32, 33) being separable.

15. An aerosol-delivery system (10) according to claim 14 as dependent on claim 13, wherein said second housing (33) has an inlet (50), said first housing (32) has an outlet, and said air-flow pathway (44a, 44b) extends to said inlet (50) and said outlet.
